# EUROPEAN PATENT APPLICATION

(11) **EP 1 746 167 A1**
(43) Date of publication of application: **24.01.2007**
(21) Application number: 05291551.9
(22) Date of filing: 20.07.2005
(51) Int. Cl.: C12Q 1/37

(54) **Method for the identification of compounds susceptible to inhibit inflammation**

(71) Applicant: Apoxis SA, 1066 Epalinges (CH)
(72) Inventor: Tschopp, Jurg, 1066 Epalinges (CH); Martinon, Fabio, 1004 Lausanne (CH)
(74) Representative: Warcoin, Jacques

(57) **Abstract**

The present invention concerns a method for the identification of compounds susceptible to inhibit the activity of the inflammasome comprising contacting at least one compound to be screened with an assay appropriate to selectively detect and quantify the inflammasome formation in vitro. The invention also concerns the use of the compound or group of compounds being selected by the method according to the invention in the development of drugs to be used in the treatment of conditions where control of inflammation is needed.

## Description

The present invention concerns a new method for the identification of compounds susceptible to inhibit inflammation.

Inflammation is a general term for the local accumulation of fluid, plasma proteins, and white blood cells that is initiated when a group of cells or an organism is put under stress, by physical injury such as DNA damages, infection, or a local immune response. This is also known as an inflammatory response. The cells that invade tissues undergoing inflammatory responses are often called inflammatory cells or an inflammatory infiltrate and help cells or organisms to improve their conditions as a response to the stress.

The inflammatory response is highly regulated. Deregulation of inflammation can provoke inflammatory diseases. Inflammation entails the four well-known symptoms redness, heat, tenderness/pain, and swelling that characterize so many common diseases and conditions. Chronic inflammatory diseases, such as rheumatoid arthritis, inflammatory bowel disease, systemic lupus erythematosus, multiple sclerosis, and type 1 diabetes, affect almost half a billion of people. Many of these diseases are debilitating and are becoming increasingly common in our aging society.

One of the key factors that initiate inflammation is the cytokine IL-1β (Dinarello, 2005). It is produced as an inactive precursor protein in many cells under stress conditions and requires proteolytic processing for its activity (Martinon and Tschopp, 2004). The complex, which senses the stress, is called the inflammasome. The inflammasome is a multiprotein complex responsible for the activation of the caspases 1 and 5 thereby leading to the processing and activation of the pro-inflammatory cytokines IL-1β and IL-18 (Martinon et al., 2002). Processing causes secretion of the active cytokines and initiates a potent inflammatory response.

Two types of inflammasomes have been identified to date (Martinon and Tschopp, 2004). The NALP1-inflammasome, which is composed of NALP1/ASC/Caspase-1/Caspase-5 and the NALP2/3-inflammasomes that contain, in addition to NALP2 or NALP3, CARDINAL/ASC/Caspase-1. All the inflammasome components assemble in the cytoplasm. Intriguingly, after activation of the inflammasome, several components, namely caspase-1, - 5 and ASC, are secreted along with the IL-1β.

Little is known about the natural stimuli that lead to the assembly and activation of the inflammasomes. Similar to Toll-like receptors, activation of the inflammasome is thought to occur through the recognition of pathogen-associated patterns (PAMPs) by the Leucine-Rich Repeats (LRR) present in the NALP proteins. The only bacterial component that is currently known to activate the (NALP3)inflammasome is muramyl dipeptide (MDP), a component of peptidoglycans, which is a cell wall component present in both Gram-positive and Gram-negative bacteria.

Excessive or uncontrolled IL-β production is harmful to the host and is therefore tightly controlled (Dinarello, 2005). Several proteins such as COP, PI-9, Pyrin and ICEBERG are thought to regulate the inflammasome activity, through interference with either the recruitment or the activity of caspase-1.

Mutations in the gene coding for NALP3, *CIAS1,* have been associated with several autoinflammatory disorders such as Muckle-Wells syndrome, familial cold urticaria and CINCA (Chronic Infantile Neurological Cutaneous and Articular autoinflammatory disease) (Martinon and Tschopp, 2004). These disorders are characterized by recurrent episodes of fever and serosal inflammation, due to increased production of IL-1β. They can be successfully treated with the natural IL-1 inhibitor IL-1ra (Anakinra).

Therefore, controlling the factors involved in the formation and destruction of the inflammasome will allow to treat pathological inflammatory responses.

The present invention concerns therefore a new method for the identification of compounds susceptible to inhibit inflammasome formation and activity.

The method of the invention comprises contacting at least one compound to be screened with an assay appropriate to selectively detect and quantify the inflammasome formation and activity in vitro.

In a preferred embodiment, the assay comprises measuring activation of caspase-1 after inflammasome formation. Activation of caspase-1 can be measured and quantified by various means known to the skilled person, such as Western blot monitoring with appropriate antibodies, or measurement of cleavage of caspase-1 substrates, preferably labeled substrates such as substrates labeled with fluorogenic markers.

A preferred assay comprises in vitro activating the inflammasome by cell lysis in an appropriate medium comprising the means necessary to measure inflammasome formation and activity as disclosed above and in the examples below.

Performing the assay under specific conditions in the absence of compound to be screened will give a control value for inflammasome formation and activity under these specific conditions. Performing the same assay under the same conditions but in the presence of a compound or a group of compounds to be screened will give a value associated to each of the compounds or group of compounds under the same specific conditions.

Comparing the compounds associated values with the control value will allow the person skilled in the art to identify whether the screened compound inhibit inflammasome formation and activity.

Those compounds inhibiting inflammasome formation and activity are potential drugs susceptible to be used in the treatment of inflammation diseases such as rheumatoid arthritis, inflammatory bowel disease, ulcerative colitis, psoriasis, systemic lupus erythematosus, multiple sclerosis, and type 1 diabetes,

Compounds according to the invention are any compounds susceptible to be screened for their activities, including natural extracts of plants, animals or microorganisms, proteins, antibodies or small molecules.

These compounds are screened either in a pure form or in mixtures with other compounds.

The present invention also concerns a method for screening a compound library to select among the compounds of the library those inhibiting inflammasome formation and activity.

The compound or group of compounds being selected by the method according to the invention are then used in the development of drugs to be used in the treatment of conditions where control of inflammation is needed, such as the diseases listed above.

The method according to the invention may be used either *ab initio* for the selection of new compounds (hits) going to be developed as new drugs, or late in the process of drug design to help selecting a lead compound based on its properties on inflammasome formation and activity and knowledge of mode of action.

The present invention also concerns such compounds which have been selected with a method according to the invention and being used in the treatment of the above diseases.

Other characteristics of the method according to the present invention may be found in the disclosure of specific examples below.

### Description of the drawings

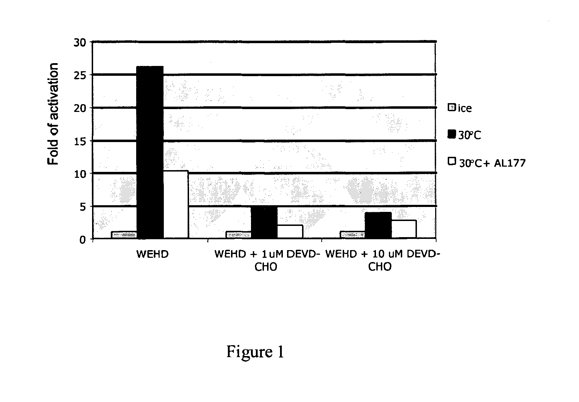

**Figure 1**: Inflammasome activity as detected by ac-WEHD-AMC cleavage assay. Activation is presented as fold of activation of the sample incubated at 4°C. The inflammasome antagonist antibody AL177 (Martinon et al. 2002) was used as a positive control of specific inhibition of the inflammasome. The effect of two concentrations of the caspase inhibitor DEVD-CHO are also shown..

### Examples

### 1) Preparation of cell extracts for Inflammasome assay.

THP1 cells (ATCC TIB-202) were grown in suspension in roller bottles in RPMI 1640 medium supplemented with 10% heat inactivated fetal bovine serum, 50µM β-mercaptoethanol and penicillin/streptomycin (100µg/ml of each) to a density of ~1.5 x10⁶ cells/ml, subcultured weekly. The cytosolic fraction were isolated from THP1 lysats as described previously (Martinon et al. 2002). Briefly, the cells were washed in phosphatebuffered saline, swollen in 3 vol (relative to cell volume) of ice-cold hypotonic buffer W (20 mM Hepes-KOH [pH 7.5], 10 mM KCl, 1.5 mM MgCl₂, 1 mM sodium EDTA, 1 mM sodium EGTA, and 0.1 mM PMSF), supplemented with a protease inhibitor cocktail (Roche). After leaving on ice for 15 min, cells were broken by passing 15 times through a G22 needle. After centrifugation at 4°C, the resulting supernatants were filtered (filter 0.45µm diameter pores) on ice and directly used for the in vitro fluorometric inflammasome activity assay, or frozen at -70°C for later analysis.

### 2) Determination of the inflammasome activity with fluorogenic caspases substrates.

The specificity of the proteolytic cleavage done by caspases is dictated by a recognition sequence of four amino acids (P4, P3, P2 & P1). The cleavage is occurring after the P1 residue which is always an aspartic acid. To measure caspase-1 (Inflammasome) activity, the fluorogenic substrate Ac-Trp-Glu-His-Asp-AMC (ac-WEHD-AMC, Alexis ; ALX-260-057; stock solution : 50mM in DMSO) was used.
To monitor inflammasome activation, 50µl of cytosolic extracts as described in point 1, were incubated on ice (basal activity) or at 30°C (inflammasome activity) for 40 minutes. in the presence of ac-WEHD-AMC (1mM final) and with or without a candidate inhibitor of the inflammasome, such as an antagonistic antibody to ASC (AL177, Alexis, ALX-210-905). The reaction was stopped by the diluting the sample 5 times with ice cold buffer W (final volume 250µl).
To determine fluorogenic activity, 100µl of the diluted samples were loaded into 96 wells plates. 100µl of buffer W was used for background determination (blank). Fluorescence was measured with a Fluorometer (Fluoroskan II, excitation at 355 nm, emission at 460 nm).

Alternatively, a 96 wells plate containing the various compounds to test was analyzed by adding 20µl/well of ice cold cytosolic lysates (as prepared in point 1) complemented with 1mM ac-WEHD-AMC, on ice, and incubated at 30°C for 40 min. 10µM of Z-VAD-fmk or DEVD-CHO (caspase inhibitors) were used in some wells as positive control of inhibition (see Figure 1). After activation the reaction was stopped by adding 80µl of ice cold buffer W. Fluorogenic activity was measured with a Fluorometer (Fluoroskan II, excitation at 355 nm, emission at 460 nm).

### References

✔ Dinarello, C. A. (2005). Blocking IL-1 in systemic inflammation. J Exp Med 201, 1355-1357.
✔ Martinon, F., Bums, K., and Tschopp, J. (2002). The Inflammasome. A Molecular Platform Triggering Activation of Inflammatory Caspases and Processing of proIL-beta. Mol Cell 10, 417.
✔ Martinon, F., and Tschopp, J. (2004). Inflammatory caspases: linking an intracellular innate immune system to autoinflammatory diseases. Cell 117, 561-574.

## Claims

1. Method for the identification of compounds susceptible to inhibit the formation and activity of the inflammasome comprising contacting at least one compound to be screened with an assay appropriate to selectively detect and quantify the inflammasome formation and activity in vitro.

2. Method as claimed in claim 1, **characterized in that** the assay comprises measuring activation of caspase-1 after inflammasome formation.

3. Method as claimed in one of claims 1 or 2, **characterized in that** the assay comprises *in vitro* activating the inflammasome by cell lysis in an appropriate medium comprising the means necessary to measure inflammasome formation.

4. Method as claimed in one of claims 1 to 3, **characterized in that** the compounds are selected among natural extracts of plants, animals or microorganisms, proteins, antibodies or small molecules.

5. Method as claimed in one of claims 1 to 4, **characterized in that** the compounds are screened either in a pure form or in mixtures with other compounds.

6. Method as claimed in one of claims 1 to 5 for screening a compound library to select among the compounds of the library those inhibiting the formation and activation of the inflammasome.

7. Use of the compound or group of compounds being selected by the method according to one of claims 1 to 6 in the development of drugs to be used in the treatment of conditions where control of inflammation is needed.
